# FASCICULE DE BREVET EUROPEEN

(11) **EP 1 993 462 B1**
(45) Date de publication et mention de la délivrance du brevet: **27.10.2010**
(21) Numéro de dépôt: 07718029.7
(22) Date de dépôt: 19.01.2007
(51) Int. Cl.: A61B 19/08

(54) **PERFECTIONNEMENTS AUX CHAMPS OPERATOIRES A FENETRE**
VERBESSERTE OP-ABDECKTÜCHER MIT FENSTER
IMPROVEMENTS TO OPERATING DRAPES WITH A WINDOW

(30) Priorité: 19.01.2006 FR 0600482
(43) Date de publication de la demande: 26.11.2008
(73) Titulaire: VYGON, F-95440 Ecouen (FR)
(72) Inventeur: CARREZ, Jean Luc, F-95440 Ecouen (FR); DALLE, Valéry, F-60270 Gouvieux (FR); HOCQ, Xavier, F-59530 Viller Pol (FR); LESTOQUOY, Patrick, F-59551 Attiches (FR)
(74) Mandataire: Le Forestier, Eric
(86) Numéro de dépôt international: PCT/FR2007/000103
(87) Numéro de publication internationale: WO 2007/083032

(56) Documents cités:
- EP-A- 0 494 413
- WO-A-99/16377
- GB-A- 2 324 244
- US-A- 5 002 070
- US-A- 6 129 085
- US-B1- 6 345 622

## Description

L'invention concerne un champ opératoire imperméable qui comporte une fenêtre au travers de laquelle l'opérateur peut accéder au site opératoire et introduire dans le corps un tube ou un fil éventuellement prolongé par une ligne de perfusion ou autre ligne qui doit rester en place alors que le champ doit être retiré.

Pour retirer le champ malgré la présence du tube du fil ou de la ligne qui traversent la fenêtre il est connu de découper le champ au moyen d'un instrument ou de le déchirer le champ au moment voulu pour le retrait, jusqu'à ouvrir latéralement la fenêtre et ces opérations compliquent le travail de l'opérateur et constituent un risque pour le tube, le fil ou la ligne.

Pour faciliter ces opérations, il a été proposé de munir le champ d'amorces de découpe ou de déchirement, comme décrit par exemple dans la publication EP 1 009 318.

Ces mesures ne facilitent que dans une mesure limitée le découpage du champ et n'éliminent pas les risques.

La présente invention vise à assurer une élimination facile et sure du champ sans l'intervention d'un outil et sans recourir à un matériau déchirable pour la constitution du champ.

La publication WO 99/16377 décrit des champs à fenêtre dans lesquels sont constitués des lignes qui se rejoignent sur la fenêtre et qui permettent de déchirer le champ pour l'adapter plus facilement à la forme du visage, et notamment des sourcils et paupières, car il s'agit d'un champ utilisé en ophtalmologie.

La publication expose que ces lignes sont constituées par des perforations ou des indentations ou des affaiblissements de la feuille. En fait, la seule méthode effectivement décrite est la découpe de perforations avec un couteau rotatif.

La problématique de la présente invention est d'avoir un champ avec une fenêtre pour l'opération, et de pouvoir le séparer ensuite en deux parties pour l'enlever malgré les tubulures qui sortent du site opératoire par la fenêtre et sont connectées à des appareils.

Un champ avec une ligne de pointillés prédécoupés peut permettre cette séparation, mais il n'est pas imperméable autour du site opératoire. Or, il doit l'être pour des questions d'infections possibles.

Des indentations ou un affaiblissement thermique fragilisent le champ et favorisent la formation accidentelle de perforations.

Le document EP 0 494 413 décrit un champ constitué par une nappe imperméable, formée par deux feuilles imperméables maintenues dans le prolongement l'une de l'autre, et présentant une fenêtre d'accès au site opératoire partagée entre les deux feuilles.

La présente invention permet d'assurer l'imperméabilité sans fragiliser la feuille et de séparer la feuille en deux sans l'aide d'un outil coupant.

On y parvient selon l'invention avec un champ opératoire constitué par une nappe imperméable qui présente une fenêtre (3) d'accès au site opératoire, **caractérisé en ce que** la nappe est constituée de deux feuilles imperméables (10, 20) maintenus dans le prolongement l'une de l'autre et de façon étanche par un adhésif qui permet de séparer les feuilles par pelage et en ce que la fenêtre (3) est partagée entre les deux feuilles.

Dans de modes de réalisation préférés, le champ présente également une ou plusieurs des caractéristiques suivantes :
- les deux feuilles présentent deux bords jointifs maintenus par un ruban imperméable placé sous lesdits bords et auxquels lesdits bords sont fixés par ledit adhésif ;
- les deux feuilles présentent deux bords en superposition et ledit adhésif est disposé entre lesdits bords ;
- lesdits bords sont rectilignes ;
- ladite fenêtre est partagée sensiblement par moitié entre les deux feuilles ;
- lesdites feuilles sont rectangulaires ;
- des moyens absorbants sont prévus autour de la fenêtre ;
- les moyens absorbants sont constitués par deux bandes absorbantes qui sont complémentaires pour former un cadre autour de fenêtre et qui sont collées au champ sauf à des endroits où elles se chevauchent en sorte qu'elles restent libre à ces endroits ;
- le champ comporte plusieurs fenêtres superposées de tailles décroissantes réalisées dans des masques successifs pelables latéralement.

On décrira ci-après des exemples de réalisation d'un champ opératoire selon l'invention, en référence aux figures jointes sur lesquelles :
- la figure 1 représente la face de dessous d'une réalisation d'un champ conforme à l'invention, c'est-à-dire la face qui sera au contact de la peau, avant réalisation de la fenêtre ;
- la figure 2 représente le champ de la figure 1 après réalisation d'une fenêtre ;
- la figure 3 représente le champ de la figure 2 après application d'un adhésif ;
- la figure 4 représente la face de dessus du champ après mise en place de bandes absorbantes ;
- la figure 5 représente les fenêtres d'un champ à plusieurs fenêtres superposées ;
- la figure 6 représente la face de dessous d'une variante d'un champ conforme à l'invention, avant réalisation de la fenêtre et
- la figure 7 représente le champ de la figure 6 après réalisation de la fenêtre.

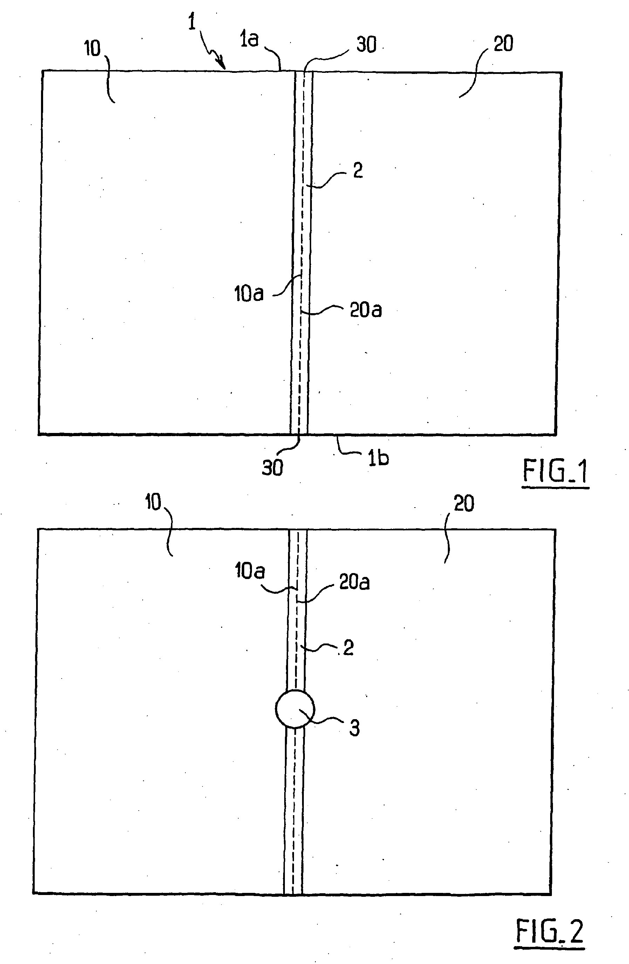

Le champ (1) représenté sur la figure 1 est constitué de deux feuilles imperméables (10,20) rectangulaires, par exemple de 100 x 70 cm dont deux bords (10a, 20a) sont jointifs. Sous ces bords, les feuilles sont fixées adhésivement à une bande imperméable (2), par exemple de 5 à 10 cm de largeur par un adhésif permettant de détacher les feuilles de la bande par pelage.

Dans le champ est pratiqué (figure 2), un trou qui constitue une fenêtre (3) qui est partagée entre les deux feuilles, par exemple en deux parties sensiblement égales.

Cette fenêtre a toute forme et toutes dimensions voulues, par exemple circulaire (figure 2) ou ovale.

De l'adhésif (4) est appliqué autour du trou sur la face de dessous du champ et cet adhésif comme le trou est protégé provisoirement par un papier siliconé (5) qui sera retiré pour la fixation du champ autour du site opératoire.

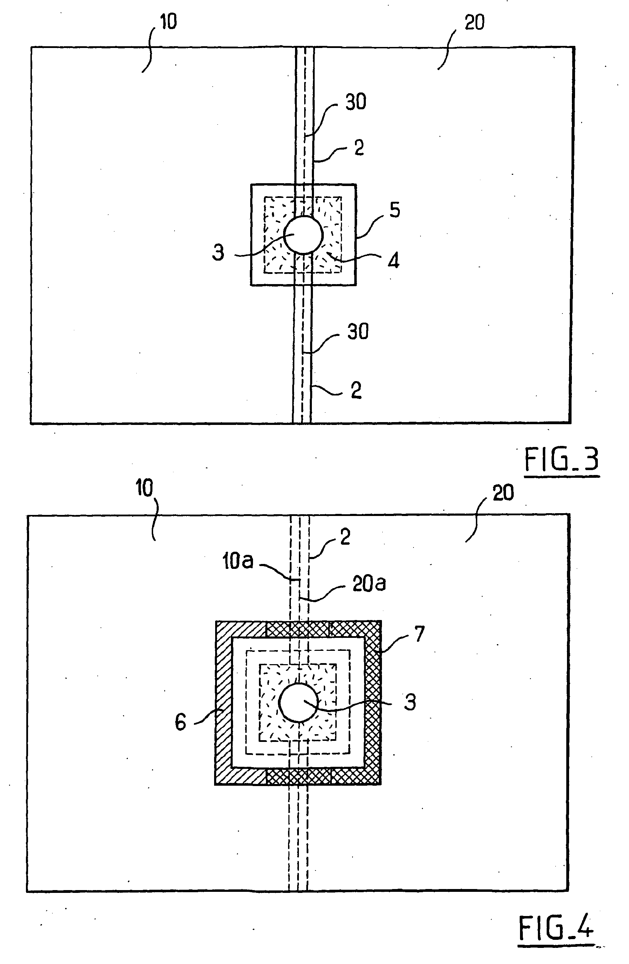

Sur la face de dessus du champ (figure 4) sont fixées adhésivement autour du trou (3) deux bandes en U (6,7) en matériau absorbant, par exemple en non-tissé ; les deux bandes sont placées en opposition en sorte que leurs ailes se chevauchent sur la ligne de coupure et ne soit fixée à l'endroit du chevauchement ni au champ entre elles.

Les bandes en U constituent un carré intérieur d'environ 40 cm de côté et un carré extérieur de 50 à 60 cm de côté. A l'intérieur de carré de 40 cm tous les matériaux hors papier siliconé sont de préférence transparents.

Le champ peut comporter de façon en soi connue plusieurs fenêtres superposées dont la taille est décroissante depuis la fenêtre initiale du champ jusqu'à la fenêtre située le plus en dessus.

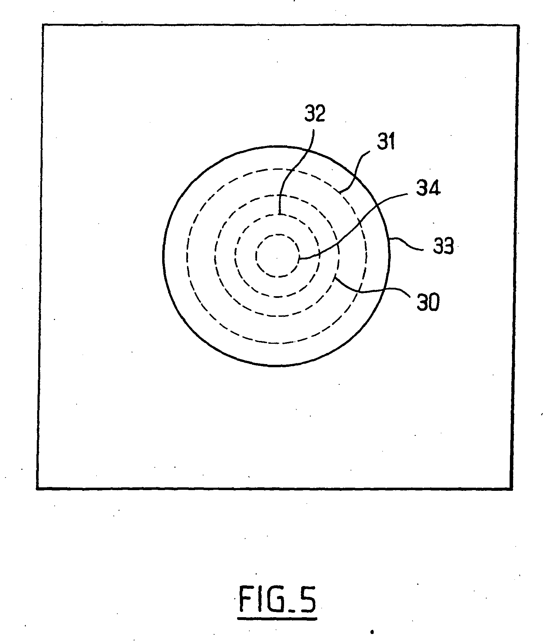

La figure 5 représente pour l'exemple un champ qui comporte un fenêtre initiale (30) par exemple de 15cm de diamètre, recouverte par un premier masque adhésif pelable (31) qui comporte lui-même une fenêtre (32), par exemple de 10 cm de diamètre laquelle est recouverte par un deuxième masque adhésif pelable (33) pourvu d'une fenêtre (34), par exemple de 5 cm de diamètre. Les masques sont fendus pour permettre un pelage latéral.

A la pose du champ, on enlève le papier siliconé. On applique le champ sur la peau du patient. Pour cela, on peut voir à travers le carré transparent.

On procède à l'opération et à la mise en place des lignes permanentes (perfusion, mesure de pression artérielle, et autres), puis on sépare l'une des feuilles de la base adhésive en la pelant latéralement jusqu'au trou du site opératoire, on tire latéralement le champ autour des lignes, les bandes en U s'écartent aussi sans offrir de résistance autour des lignes installées.

On décolle le champ de la peau du patient avant de le tirer latéralement.

Les bandes en U servent à absorber les liquides lors de l'opération, le carré de non tissé peut être orienté de manière à éviter d'avoir en position basse du champ les côtés où les bandes se chevauchent.

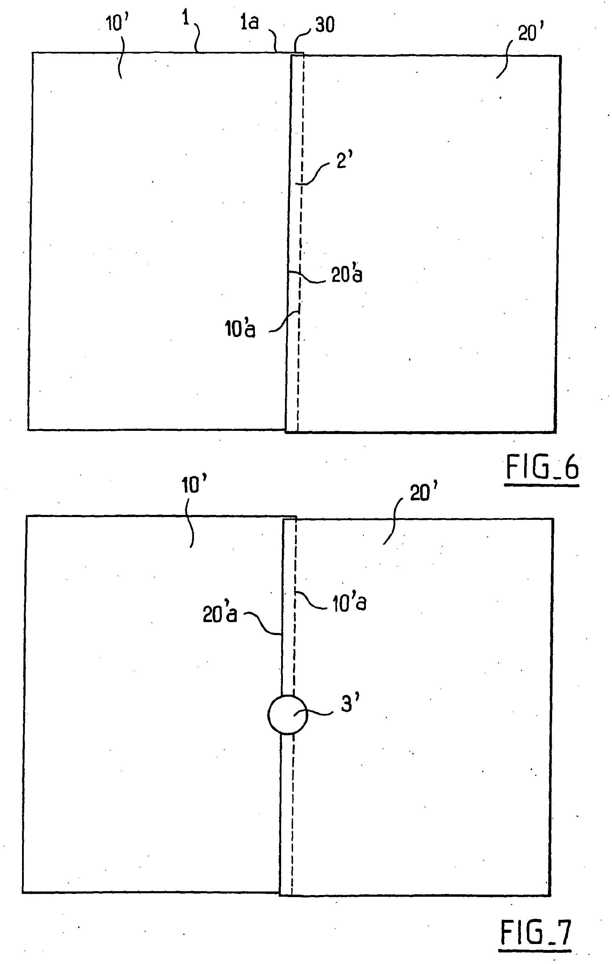

Dans une variante de réalisation (figures 6 et 7) les deux feuilles (10', 20') du champ présentent deux bords (10'a, 20'a, 10'a, 20'a) en superposition et un adhésif permettant le pelage est disposé entre les bords superposés. L'adhésif peut être porté par un ruban (2). Le recouvrement des deux feuilles est par exemple d'une largeur de 5cm. La perforation (3')est pratiquée comme précédemment.

L'invention n'est pas limitée aux réalisations qui ont été décrites.

## Revendications

1. Champ opératoire constitué par une nappe imperméable, constituée de deux feuilles imperméables (10, 20) maintenues dans le prolongement l'une de l'autre, qui présente une fenêtre (3) d'accès au site opératoire, partagée entre les deux feuilles, **caractérisé en ce que** les deux feuilles imperméables (10, 20) sont maintenues de façon étanche par un adhésif qui permet de séparer les feuilles par pelage.

2. Champ opératoire selon la revendication 1 dont les deux feuilles présentent deux bords jointifs (10a, 20a) maintenus par un ruban imperméable (2) placé sous lesdits bords et auxquels lesdits bords sont fixés par ledit adhésif.

3. Champ opératoire selon la revendication 1 dont les deux feuilles (10', 20') présentent deux bords (10'a, 20'a) en superposition et en ce que ledit adhésif est disposé entre lesdits bords.

4. Champ opératoire selon la revendication 1 ou 2 dont lesdits bords (10a, 20a) sont rectilignes.

5. Champ opératoire selon l'une des revendications 1 à 4 dont ladite fenêtre (3) est partagée sensiblement par moitié entre les deux feuilles (10,20).

6. Champ opératoire selon l'une des revendications 1 à 5 dont lesdites feuilles (10, 20) sont rectangulaires.

7. Champ selon l'une des revendications 1 à 6 et qui comprend des moyens absorbants (6,7) autour de la fenêtre (3) qui sont fendus pour un pelage latéral.

8. Champ selon la revendication 7 et dont les moyens absorbants sont constitués par deux bandes absorbantes (6, 7) qui sont complémentaires pour former un cadre autour de la fenêtre (3) et qui se chevauchent, les deux bandes étant collées au champ sauf à des endroits où les deux bandes se chevauchent en sorte qu'elles restent libres à ces endroits.

9. Champ selon la revendication 8 et dans lequel les bandes absorbantes (6, 7) sont conformées en U.

10. Champ selon l'une des revendications 1 à 9 et qui comporte plusieurs fenêtres (30, 32, 34) superposées de tailles décroissantes réalisées dans des masques successifs (31, 32) pelables qui sont fendus pour un pelage latéral.

## Claims

1. An operating drape composed of an impermeable web composed of two impermeable sheets (10', 20) held in extension of each other, which has a window (3) providing access to the operating site shared between the two sheets, **characterised in that** the two impermeable sheets (10, 20) are held in a sealed manner by an adhesive which allows separation of the sheets by peeling apart.

2. The operating drape as claimed in Claim 1, in which the two sheets have two joined edges (10a, 20a) held by an impermeable ribbon (2) placed under said edges, and to which said edges are attached by said adhesive.

3. The operating drape as claimed in Claim 1, in which the two sheets (10', 20') have two superimposed edges (10'a, 20'a), and in that said adhesive is located between said edges.

4. The operating drape as claimed in Claim 1 or 2, in which said edges (10a, 20a) are rectilinear.

5. The operating drape as claimed in any one of Claims 1 to 4, in which said window (3) is shared substantially equally between the two sheets (10, 20).

6. The operating drape as claimed in any one of Claims 1 to 5, in which said sheets (10, 20) are rectangular.

7. The drape as claimed in any one of Claims 1 to 6, which includes absorbent means (6,7) around the window (3) which are split for peeling apart laterally.

8. The drape as claimed in Claim 7, in which the absorbent means are composed of two absorbent strips (6, 7) which are complementary so as to form a frame around the window (3), and which overlap, the two strips being affixed to the drape except at positions where the two strips overlap, so that they remain free at these positions.

9. The drape as claimed in Claim 8, in which the absorbent strips (6, 7) are U-shaped.

10. The drape as claimed in any one of Claims 1 to 9, which includes several superimposed windows (30, 32, 34) of decreasing size created in successive peelable masks (31, 32) which are split for peeling apart 20 laterally.

## Patentansprüche

1. Abdecktuch, das aus einem wasserdichten Tuch besteht, das aus zwei wasserdichten Bögen (10, 20) besteht, die in gegenseitiger Verlängerung gehalten werden, das ein Zugangsfenster (3) zur Operationsstelle aufweist, das zwischen den zwei Bögen geteilt ist, **dadurch gekennzeichnet, dass** die zwei wasserdichten Bögen (10, 20) von einem Haftstoff dicht gehalten werden, der es erlaubt, die Bögen durch Abziehen zu trennen.

2. Abdecktuch nach Anspruch 1, dessen zwei Bögen zwei fugendichte Ränder (10a, 20a) aufweisen, die von einem wasserdichten Streifen (2) gehalten werden, der unter den Rändern platziert ist und an dem die Ränder durch den Haftstoff befestigt sind.

3. Abdecktuch nach Anspruch 1, dessen zwei Bögen (10', 20') zwei übereinander liegende Ränder (10'a, 20'a) aufweisen und **dadurch**, dass der Haftstoff zwischen den Rändern angeordnet ist.

4. Abdecktuch nach Anspruch 1 oder 2, dessen Ränder (10a, 20a) geradlinig sind.

5. Abdecktuch nach einem der Ansprüche 1 bis 4, dessen Fenster (3) etwa hälftig zwischen den zwei Bögen (10, 20) geteilt ist.

6. Abdecktuch nach einem der Ansprüche 1 bis 5, dessen Bögen (10, 20) rechteckig sind.

7. Tuch nach einem der Ansprüche 1 bis 6 und das um das Fenster (3) absorbierende Mittel (6, 7) umfasst, die für ein seitliches Abziehen geschlitzt sind.

8. Tuch nach Anspruch 7 und dessen absorbierende Mittel von zwei absorbierenden Streifen (6, 7) gebildet werden, die komplementär sind, um einen Rahmen um das Fenster (3) zu bilden und die sich überlappen, wobei die zwei Streifen auf dem Tuch verklebt sind außer an Stellen, wo sich die zwei Streifen derart überlappen, dass sie an diesen Stellen frei bleiben.

9. Tuch nach Anspruch 8 und bei dem die absorbierenden Streifen (6, 7) als U ausgebildet sind.

10. Tuch nach einem der Ansprüche 1 bis 9 und das mehrere übereinander liegende Fenster (30, 32, 34) abnehmender Größe aufweist, die in aufeinander folgenden abziehbaren Masken (31, 32) realisiert sind, die für ein seitliches Abziehen geschlitzt sind.
